# EUROPEAN PATENT SPECIFICATION

(11) **EP 0 433 299 B1**
(45) Date of publication and mention of the grant of the patent: **06.05.1998**
(21) Application number: 89909128.4
(22) Date of filing: 02.08.1989
(51) Int. Cl.: A61K 39/02, A61K 39/08, A61K 39/10, A61K 39/114, A61K 35/24

(54) **TREATMENT OF GASTRO-INTESTINAL DISORDERS**
BEHANDLUNG VON GASTRO-INTESTINALEN KRANKHEITEN
TRAITEMENT DE TROUBLES GASTRO-INTESTINAUX

(30) Priority: 02.08.1988 AU 9613/88; 21.04.1989 AU 3837/89
(43) Date of publication of application: 26.06.1991
(73) Proprietor: Gastro Services Pty. Limited (ACN 002 994 890), Five Dock New South Wales 2046 (AU)
(72) Inventor: Gastro Services Pty. Limited (ACN 002 994 890), Five Dock New South Wales 2046 (AU)
(74) Representative: Cresswell, Thomas Anthony
(86) International application number: AU8900328
(87) International publication number: WO9001335

(56) References cited:
- AU-B- 3 758 085
- AU-B- 5 329 879
- DE-A- 2 134 179
- FR-A- 2 244 464
- FR-M- 0 001 275
- FR-M- 0 002 427
- FR-M- 0 002 828
- FR-M- 0 005 528
- GB-A- 1 271 674
- US-A- 3 713 836
- US-A- 4 335 107
- PATENT ABSTRACTS OF JAPAN, C-46, p. 141/

## Description

The present invention relates to the use of medicaments in methods of treating diseases in humans, in particular to the treatment of chronic disorders associated with the presence of abnormal or an abnormal distribution of microflora in the gastrointestinal tract.

There are large numbers of patients suffering from gastro-intestinal symptoms referrable to the lower small bowel and large bowel which to date have eluded explanation. These disorders include irritable bowel syndrome (IBS) or spastic colon, idiopathic ulcerative colitis, mucous colitis, collagenous colitis, Crohn's disease, inflammatory bowel disease in general, microscopic colitis, antibiotic-associated colitis, idiopathic or simple constipation, diverticular disease, and AIDS enteropathy. Pathophysiology of these disorders eludes logical explanation in spite of decades of research and millions of dollars of research funds. A common underlying factor shared by all these disorders observed by the present inventor is their onset following some extraneous invading infection. In all the disorders, the infection cannot be demonstrated due to our inability to detect infecting agents whose cultural characteristics are unknown to medical science.

Circumstantial evidence which suggests that these disorders are "infection-related" includes:
(a) onset following a gastro-intestinal infection which failed to completely resolve;
(b) transient improvement with use of certain antibiotics, but recurrence upon cessation of antibiotics;
(c) transient improvement following orthostatic lavage prior to colonoscopy and;
(d) transient symptom improvement with use of "colonic" irrigation.

It is impractical to use long-term antibiotic therapy (with its associated complications) in such patients since cure is not obtained with its use. Furthermore, chronic gut infections with recognised, specific pathogens such as Clostridium difficile, Yersinia enterocolitica or Campylobacter jejuni/coli are not eradicated with antibiotics. Some previous attempts have been made to alter the enteric microflora in order to eradicate such chronic infections. These measures nevertheless indicate that alteration of bacterial flora may effect dramatic clinical improvement in conditions characterized by chronic, resistant enterocolitic infection. However there remain many chronic disorder of uncertain aetiology or causation, which are resistant to cure by current therapeutic techniques.

By way of illustration, Australian specification AU-B-53298/79 (Nestle S.A.) describes microcapsules containing microorganisms of the genus Lactobacillus and/or Bifidobacterium. It is stated that the intestinal flora is made up of more than 80% of lactobacilli and bifidobacteria and the described microcapsules are intended to provide such bacteria where the intestinal flora has been altered by the use of antibiotics.

United States Patent US-A-4335107 describes the use of avian intestinal microflora derived from faecal droppings of salmonella-free birds for preventing infection of poultry flocks by paratyphoid salmonella.

It is thus an object of the present invention to provide novel methods of treating various disease states related to the presence of "abnormal" microflora in the gastrointestinal tract.

It is recognised that chronic infection/infestation is the underlying pathological process in a wide range of chronic disorders such as irritable bowel syndrome, particularly when characterised by chronic abdominal pain, bloating, or excessive flatulence, together with chronic diarrhoea or alternating constipation/diarrhoea, and also in spastic colon, mucous colitis, collagenous colitis, ulcerative colitis, Crohn's colitis, microscopic colitis, idiopathic inflammatory bowel disease, antibiotic-associated colitis, idiopathic or simple constipation, diverticular disease and AIDS enteropathy.

The invention has also been found to relate to other gastrointestinal disorders of unexplained aetiology such as polyposis coli and colonic polyps, which may well be influenced by the local bowel microflora.

Furthermore, it is recognised that there is a close association between the intestine and liver disease, and the intestine and migraines and chronic fatigue syndrome, and possibly other neurological syndromes such as, multiple sclerosis, amyotrophic lateral sclerosis, myasthenia gravis, Parkinson's disease, Alzheimers disease and other degenerative disorders. Hence, it is proposed that a considerable proportion of currently unexplained diseases of the liver and nervous system of unknown aetiology may be explicable by the chronic storage of pathogens within the small/large intestine and the subsequent passage of antigenic material and pathogenic TOXINS into the portal system (liver damage) or systemic circulation (neurological conditions). Specifically, such hepato/biliary system disorders as primary biliary cirrhosis, primary sclerosing cholangitis, fatty liver of unknown aetiology, or cryptogenic cirrhosis, may be secondary to chronic pathogen carrier state in the intestine.

The links between the intestine and joint disease are also recognised. Joint diseases such as rheumatoid arthritis, the non-rheumatoid arthritidies including, ankylosing spondylitis, and Reiter's syndrome, may also be causally related to a chronic intestinal carrier state, as may other syndromes with an immune mediated component such as glomerulonephritis, haemolytic uraemic syndrome, juvenile diabetes mellitus, Behcet's syndrome, coeliac disease and dermatitis herpetiformis. Similarly, syndromes with an immune complex mediated component, such as scleroderma, systemic lupus erythematosus, mixed cryoglobulinaemia, polyarteritis, familial Mediterranean fever, amyloidosis, and the various presentations of such syndromes, together with such "idiopathic" states as chronic urticaria, may be manifestations of variations of immune regulated responses to related bowel-origin pathogens chronically shedding their antigen(s) into the circulation. Other chronic conditions such as acne, and chronic idiopathic pseudo-obstructive syndrome, may well be influenced by similar mechanisms.

For many of these syndromes present therapy offers only palliation of symptoms and/or the induction of remission of the disease process but not cure. The present inventor therefore recognised the need to find a curative therapy for these wide ranging disease processes associated with considerable morbidity.

Thus the present invention provides the use of microflora in the manufacture of a medicament for treating a human patient in whom an infection cannot be demonstrated but who has a chronic gastrointestinal or gastrointestinal-associated disorder associated with the presence in the gastrointestinal tract of abnormal microflora or an abnormal distribution of microflora, wherein the medicament is either (i) a liquid culture of *Bacteroides* and *E.coli* or (ii) a composition of fresh homologous faeces, equivalent freeze-dried and reconstituted faeces or a synthetic faecal composition, and at least a portion of the patient's existing enteric microflora is removed and substituted by an effective amount of the medicament to restore in the patient a normal healthy human faecal microflora.

In its preferred form the treatment should effect a cure of the symptoms of such disorders. The change of flora is preferably as "near-complete" as possible and the flora is replaced by viable organisms which will crowd out any remaining, original flora.

The present invention encompasses uses of microflora in the treatment of chronic disorders in humans, in whom an infection cannot be demonstrated, associated with the presence of abnormal enteric microflora. Such disorders include but are not limited to those conditions in the following categories:
i) gastro-intestinal disorders including irritable bowel syndrome or spastic colon, ulcerative colitis, mucous colitis, collagenous colitis, Crohn's disease, inflammatory bowel disease, microscopic colitis, antibiotic associated colitis, idiopathic or simple constipation, diverticular disease, AIDS enteropathy, small bowel bacterial overgrowth, coeliac disease, polyposis coli, colonic polyps, chronic idiopathic pseudo obstructive syndrome;
ii) chronic gut infections with specific pathogens including bacteria, viruses, fungi and protozoa;
iii) liver disorders such as primary biliary cirrhosis, primary sclerosing cholangitis, fatty liver or cryptogenic cirrhosis;
iv) joint disorders such as rheumatoid arthritis, non-rheumatoid arthritidies, ankylosing spondylitis, and Reiter's syndrome;
v) immune mediated disorders such as glomerulonephritis, haemolytic uraemic syndrome, juvenile diabetes mellitus, mixed cryoglobulinaemia, polyarteritis, familial Mediterranean fever, amyloidosis, scleroderma, systemic lupus erythematosus, and Behcet's syndrome;
vi) neurological syndromes such as migraine, multiple sclerosis, amyotrophic lateral sclerosis, myasthenia gravis, chronic fatigue syndrome, Parkinson's disease, Alzheimers disease and other degenerative disorders;
vii) dermatological conditions such as, chronic urticaria, acne, dermatitis herpetiformis and vasculitic disorders;

The above disorders are all characterised by their response to treatment with the method of the present invention.

Typically the change in enteric flora comprises:
(a) substantially complete removal of existing enteric flora, and
(b) introduction of an array of predetermined flora into the gastro-intestinal system, and thus in a preferred form the method of treatment comprises substantially completely changing enteric flora in patients requiring such treatment.

Furthermore, in some of these disorders a short course of antibiotics may be required to rid tissue-invasive pathogens originating in the bowel lumen. For example, in Crohn's disease, anti-tuberculosis therapy may be required for six to twelve weeks before the bowel is cleared out and the flora content exchanged for a predetermined flora.

Preferably the removal of existing enteric flora is effected by lavage of the gastro-intestinal tract. This can be effected by methods known to those skilled in the art such as ingestion of lavage solutions such as orthostatic salt and polyethylene glycol solution, enemas or small bowel intubation and lavage.

Turning to the introduction of an array of predetermined flora into the gastro-intestinal system, this can be effected by enemas or per-colonoscope, via intubation of the small bowel using for example a large bore catheter equipped with distal balloon to effect rapid passage down the jejunum, or via the oral route with enteric-coated capsules.

The predetermined array of normal bowel microflora may comprise a composition of fresh homologous faeces, equivalent freeze-dried and reconstituted faeces or a "synthetic" faecal composition.

In a preferred form the synthetic faecal composition comprises a preparation of viable flora which preferably in proportional content, resembles normal healthy human faecal flora. Suitable microorganisms may be selected from the following; Bacteroides, Bifidobacterium, Eubacteria, Fusobacteria, Propionibacteria, Lactobacilli, anaerobic cocci, Ruminococcus, E. coli, Gemmiger, Clostridium, Desulfomonas, species and, more specifically, bacteria selected from Table 1. Preferably fungi are also present such as Monilia.

The composition for introduction into the gastro-intestinal system may comprise a liquid culture of Bacteroides and E. coli.

The composition is preferably lyophilised, pulverised and powdered. It may then be infused, dissolved such as in saline, as an enema. Alternatively the powder may be encapsulated as enteric-coated capsules for oral administration. As a powder it can preferably be provided in a palatable form for reconstitution for drinking. The composition can be combined with other adjuvants such as antacids to dampen bacterial inactivation in the stomach. Acid secretion in the stomach could also be pharmacologically suppressed using H2-antagonists or omeprazole. The powder may be reconstituted also to be infused via naso-duodenal infusion.

The composition is therefore preferably in the form of:
i) an enema composition which can be reconstituted with an appropriate diluent;
ii) enteric-coated capsules, or
iii) powder for reconstitution with an appropriate diluent for naso-enteric infusion or colonoscopic infusion, or
iv) powder for reconstitution with appropriate diluent, flavouring and gastric acid suppression agent for oral ingestion.

### Best Method of Performing the Invention

Typically the invention is applicable to a patient suffering from a chronic disorder associated with the presence of abnormal microflora in the gastrointestinal tract such as irritable bowel syndrome.

In the practice of the invention the patient's existing enteric flora is removed by gastrointestinal lavage effected by ingestion of about 3 litres of a balanced salt solution with polyethylene glycol. Lavage is continued until the removal of the existing flora is as near complete as possible.

A composition of predetermined flora in the form of a liquid culture of Bacteroides and E. coli is then infused into the patient per colonoscope in an amount sufficient to replace the removed flora, and reverse the disease process. Alternatively fresh homologous faeces obtained from a disease screened donor are liquefied and mixed with unprocessed bran. The mixture is then homogenised anaerobically under CO₂ cover and infused into the patient per colonoscope.

Cure or remission of symptoms is then monitored subjectively and by assessment of stool frequency or other appropriate criteria.

Using liquid cultures of Bacteroides and E. coli the inventor has achieved total reversal of colitis, irritable bowel syndrome and constipation.

As indicated in the method of treatment aspect of the invention, a preparatory course of appropriate antibiotics may be used. For example, Septrin for chronic yersiniasis, Metronidazole for ulcerative colitis, anti-TB therapy in Crohn's disease, or Vancomycin in chronic Clostridium difficile infestations.

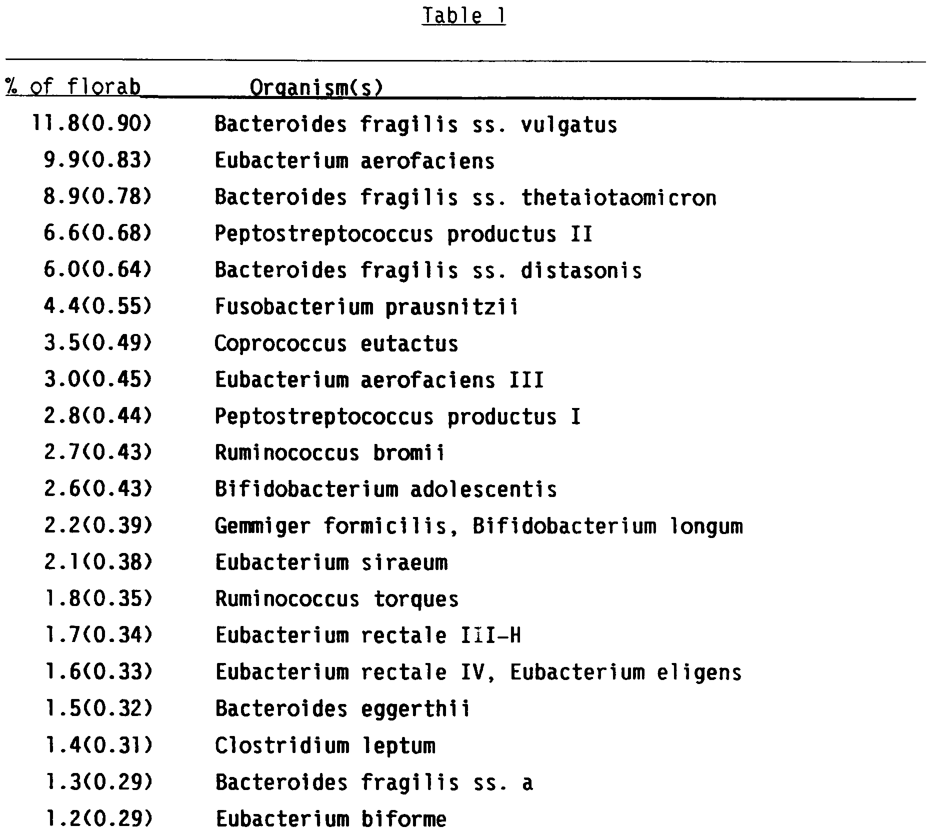

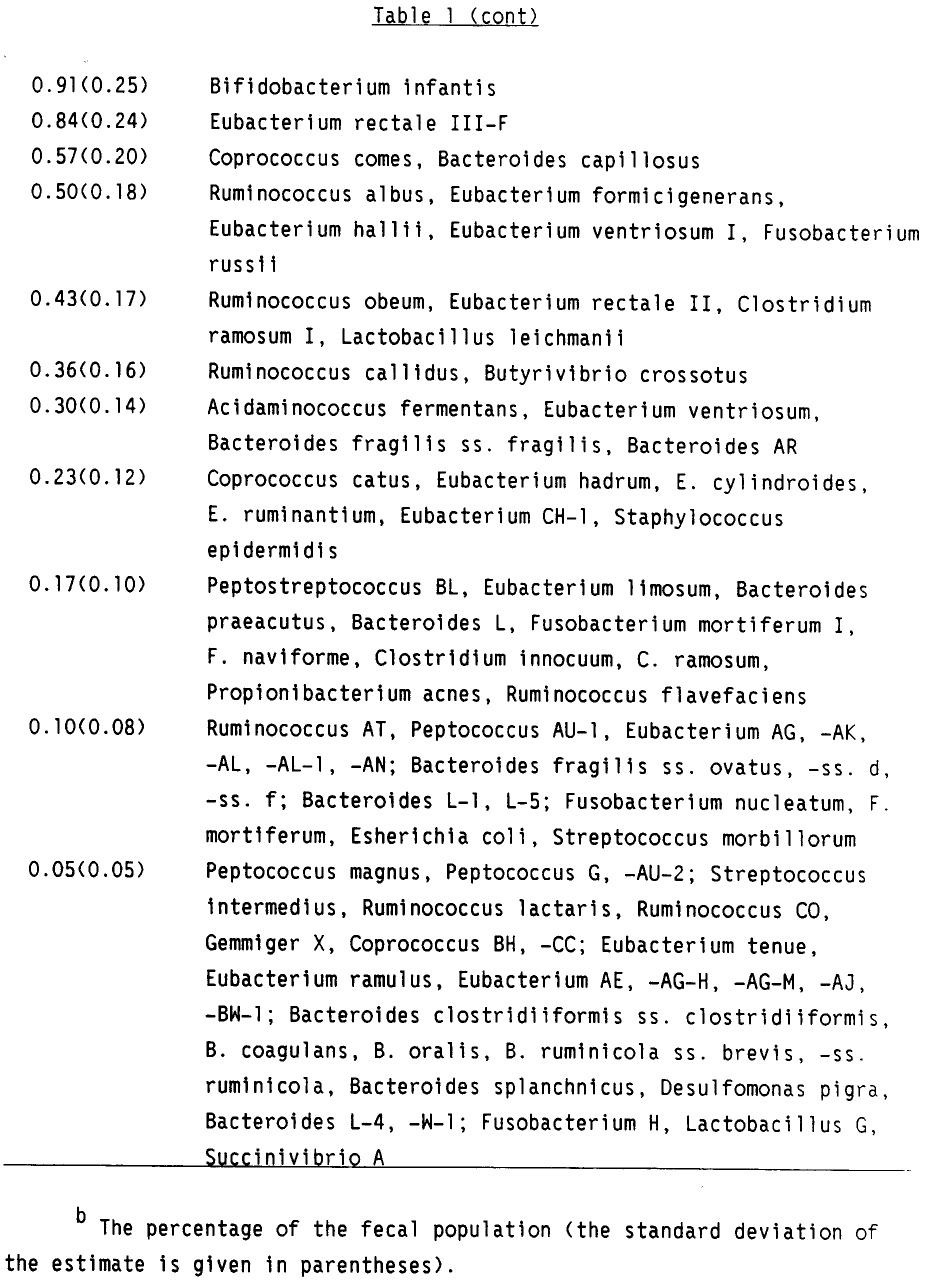

The invention will now be further described with reference to the following non-limiting examples.

### Example 1

A 28 year old male presented with bloody diarrhoea, 7 to 12 times per day, weight loss, anaemia, arthritis, marked liver-function blood test abnormalities, colonoscopic findings of "pancolitis" and histologic diagnosis of active chronic colitis. Following two years of "standard" anti-colitis therapy he improved markedly. The anaemia was corrected, he gained weight and his liver functions improved somewhat. However, he continued to experience three to four stools per day in spite of 9 x 500 mg salazopyrin tablets per day and second-nightly steriod enemas (Predsol Enemas). Furthermore, liver function tests remained elevated. Following one week of metronidazole tablets 200 mg x 4 per day, and oral ingestion of three litres balanced salt solution with polyethylene glycol (GLYCOPREP), he received fresh, liquefied homogenized human donor faeces from a disease-screened donor were infused into the patient. Prior to infusion unprocessed bran was added to the faeces and homogenization was carried out anaerobically under CO₂ cover.

Following infusion, the patient has remained well and off all treatment for three months to date. Liver function tests have returned to normal. He has one formed stool per day and has begun to put on weight. He has no arthralgia.

### Example 2

Similar methods were used to treat 55 patients suffering with either constipation, diarrhoea, abdominal pain, ulcerative colitis or Crohn's disease. Patients were treated when other forms of therapy had failed to control their symptoms. Following bowel flora alteration 20 patients were deemed "cured", 9 improved, while 26 failed to improve. The following cases illustrate "cures" using the method of the invention over a follow up period of 1 to 12 months.

### Case 1 Chronic constipation

A 31 year old female who had a history of chronic constipation commencing at birth with a frequency of once per week without laxatives. Following bowel flora alteration she has had daily or second stool frequency, without resort to laxatives and remains well at six month follow-up.

### Case 2 Chronic Pain-Predominant IBS

This 21 year old female was thoroughly investigated over a four year period for severe colickly abdominal pain, requiring frequent admissions and narcotic pain relief. Multiple investigations included endoscopy, laparoscopy and laparotomy. Five days following alteration of her bowel flora she was pain free and remains pain free at four months follow-up.

### Case 3 Chronic Diarrhoea-Predominant IBS

A 35 year old female presented with a history of several years of diarrhoea (2-10/d) and associated abdominal pain. Her only abnormal investigation was +ve stool latex test for clostridium difficile toxin. This failed to clear after a course of Vancomycin and she underwent bowel flora alteration with resolution of her chronic diarrhoea. Diarrhoea and pain have not recurred at one month follow-up.

### Case 4 Chronic Ulcerative Colitis

This 45 year old man presented with an 18 month history of ulcerative colitis and elevated liver transaminases. Pancolitis was confirmed on colonoscopy. Sulfasalazine caused a rash while olsalazine gave inadequate relief. The patient underwent exchange of bowel flora improving adequately enough to come off treatment within days. At three months he continues to feel well, has no diarrhoea and is on no medication. His liver transaminases have returned to normal. Colonoscopy is now normal and mucosal biopsies are now normal.

### Case 5 Crohn's Disease

This 31 year old man was admitted to hospital with small bowel obstruction subsequently shown on small bowel enema to be Crohn's disease of the terminal ileum. This was confirmed on terminal ileal biopsy. In spite of prednisone and sulfasalazine, hypoproteinaemia with ankle oedema was prominent presumably due to protein losing enteropathy. Three days following bowel flora alteration his ankle oedema and serum proteins returned to normal. He remains free of symptoms, off therapy 4 months later.

### Case 6 Irritable bowel/chronic fatigue syndrome

This 42 year old woman presented with symptoms of irritable bowel syndrome characterised by diarrhoea and pain, and also complained of profound tiredness. Faecal infusion reversed the bowel symptoms. In addition the profound tiredness suddenly disappeared. Similar results were obtained in 4 related cases.

## Claims

1. The use of microflora in the manufacture of a medicament for treating a human patient in whom an infection cannot be demonstrated but who has a chronic gastrointestinal or gastrointestinal-associated disorder associated with the presence in the gastrointestinal tract of abnormal microflora or an abnormal distribution of microflora, wherein the medicament is either (i) a liquid culture of *Bacteroides* and *E.coli* or (ii) a composition of fresh homologous faeces, equivalent freeze-dried and reconstituted faeces or a synthetic faecal composition, and at least a portion of the patient's existing enteric microflora is removed and substituted by an effective amount of the medicament to restore in the patient a normal healthy human faecal microflora.

2. Use according to claim 1, wherein in said treatment the existing enteric microflora is substantially completely removed.

3. Use according to claim 1 or 2, wherein the medicament comprises microorganisms selected from the group consisting of:
Bacteroides, Bifidobacterium, Eubacteria, Fusobacteria, Propionibacteria, Lactobacilli, anaerobic cocci, Ruminococcus, E.coli, Gemmiger, Clostridium, Desulfomonas, and Monilia species.

4. Use according to claim 3, wherein the medicament comprises microorganisms listed in the following group:
Bacteroides fragilis ss. vulgatus, Eubacterium aerofaciens, Bacteroides fragilis ss, thetaiotaomicron, Peptostreptococcus productus II, Bacteroides fragilis ss. distasonis, Fusobacterium prausnitzii, Coprococcus eutactus, Eubacterium aerofaciens III, Peptostreptococcus productus I,Ruminococcus bromii, Bifidobacterium adolescentis, Gemmiger formicilis, Bifidobacterium longum, Eubacterium siraeum, Ruminococcus torques, Eubacterium rectale III-H, Eubacterium rectale IV, Eubacterium eligens, Bacteroides eggerthii, Clostridium leptum, Bacteroides fragilis ss. a, Eubacterium biforme, Bifidobacterium infantis, Eubacterium rectale III-F, Coprococcus comes, Bacteroides capillosus, Ruminococcus albus, Eubacterium formicigenerans, Eubacterium hallii, Eubacterium ventriosum I, Fusobacterium russii, Ruminococcus obeum, Eubacterium rectale II, Clostridium ramosum I, Lactobacillus leichmanii, Ruminococcus callidus, Butyrivibrio crossotus, Acidaminococcus fermentans, Eubacterium ventriosum, Bacteroides AR, Bacteroides fragilis, ss. fragilis, Coprococcus catus, Eubacterium hadrum, E. cylindroides, E. ruminantium, Eubacterium CH-1, Staphylococcus epidermidis, Peptostreptococcus BL, Eubacterium limosum, Bacteroides praeacutus, Bacteroides L, Fusobacterium mortiferum I, F. naviforme, Clostridum innocuum, C. ramosum, Propionibacterium acnes, Ruminococcus flavefaciens, Ruminococcus AT, Peptococcus AU-1, Eubacterium AG, -AK, -AL, -AL-1, -AN; Bacteroides fragilis ss. ovatus, -ss. d, -ss. f; Bacteroides L-1, L-5; Fusobacterium nucleatum, F. mortiferum, Esherichia coli, Streptococcus morbillorum, Peptococcus magnus, Peptococcus G, -AU-2; Streptococcus intermedius, Ruminococcus lactaris, Ruminococcus Co, Gemmiger X, Coprococcus BH, -CC; Eubacterium tenue, Eubacterium ramulus, Eubacterium AE, -AG-H, -AG-M, -AJ, -BW-1, Bacteroides clostridiiformis ss. clostridiiformis, B. coagulans, B. oralis, B. ruminicola ss. brevis, -ss. ruminicola, Bacteroides splanchnicus, Desulfomonas pigra, Bacteroides L-4, -W-1; Fusobacterium H, Lactobacillus G, and Succinivibrio A.

5. Use according to any one of claims 1 to 4, wherein the chronic disorder is a gastrointestinal disorder selected from:
irritable bowel syndrome, spastic colon, ulcerative colitis, mucous colitis, collagenous colitis, Crohn's disease, inflammatory bowel disease, microscopic colitis, antibiotic associated colitis, idiopathic or simple constipation, diverticular disease, AIDS enteropathy, small bowel bacterial overgrowth, coeliac disease, polyposis coli, colonic polyps, chronic idiopathic pseudo obstructive syndrome and chronic enteric infections.

6. Use according to claim 5 wherein the chronic enteric infection is caused by a bacteria, virus, protozoa or fungi.

7. Use according to any one of claims 1 to 4, wherein the chronic disorder is a liver disorder such as primary biliary cirrhosis, primary sclerosing cholangitis, fatty liver or cryptogenic cirrhosis.

8. Use according to any one of claims 1 to 4, wherein the chronic disorder is a joint disorder such as rheumatoid arthritis, a non-rheumatoid arthritidies, ankylosing spondylitis, or Reiter's syndrome.

9. Use according to any one of claims 1 to 4, wherein the chronic disorder is an immune mediated disorder such as glomerulonephritis, haemolytic uraemic syndrome, juvenile diabetes mellitus, mixed cryoglobulinaemia, polyarteritis, familial Mediterranean fever, amyloidosis, scleroderma, systemic lupus erythematosus, or Behcet's syndrome.

10. Use according to any one of claims 1 to 4, wherein the chronic disorder is a neurological disorder such as migraine, multiple sclerosis, amyotrophic lateral sclerosis, myasthenia gravis, chronic fatigue syndrome, Parkinsons's disease, or Alzheimer's disease, or wherein the chronic disorder is a dermatological disorder such as chronic urticaria, acne, dermatitis herpetiformis or a vasculitic disorder.

11. Use according to any one of claims 1 to 4 wherein the chronic disorder is necrotising enterocolitis.

12. Use according to any one of claims 1 to 11, wherein the removal of existing flora is carried out by gastrointestinal lavage with orthostatic salt solution and polyethylene glycol solution.

13. Use according to claim 12, wherein gastrointestinal lavage is effected by enemata, oral ingestion or per enteral infusion.

14. Use according to any one of claims 1 to 13, wherein the medicament is introduced into the patient's gastrointestinal system by ingestion or by per enteral infusion.

15. Use according to any one of claims 1 to 14, wherein an effective amount of at least one antibiotic is administered prior to the removal of the existing flora.

## Patentansprüche

1. Verwendung von Mikroflora bei der Herstellung eines Arzneimittels zum Behandeln eines menschlichen Patienten, bei dem eine Infektion nicht nachgewiesen werden kann, der aber eine chronische Magen-Darm- oder eine mit Magen und Darm zusammenhängende Störung aufweist, die mit der Anwesenheit abnormaler Mikroflora oder einer abnormalen Mikrofloraverteilung im Magen-Darm-Trakt verbunden ist, wobei das Arzneimittel entweder (i) eine flüssige Kultur von *Bacteroides* und *E. coli* oder (ii) eine Zusammensetzung aus frischen, homologen Fäzes, gleichwertigen gefriergetrockneten und wiederhergestellten Fäzes oder eine synthetische Fäzeszusammensetzung ist und mindestens ein Teil der bei dem Patienten vorhandenen Darmmikroflora entfernt und durch eine wirksame Menge des Arzneimittels unter Wiederherstellen einer normalen, gesunden, menschlichen Fäzes-Mikroflora bei dem Patienten ersetzt wird.

2. Verwendung gemäß Anspruch 1, wobei bei der Behandlung die bestehende Darmmikroflora im wesentlichen vollständig entfernt wird.

3. Verwendung gemäß Anspruch 1 oder 2, wobei das Arzneimittel Mikroorganismen umfaßt, die aus der aus *Bacteroides, Bifidobacterium*, *Eubacteria*, *Fusobacteria*, *Propionibacteria*, *Lactobacilli*, anaeroben Kokken, *Ruminococcus*, *E. coli*, *Gemmiger*, *Clostridium*, *Desulfomonas* und *Monilia*-Arten bestehenden Gruppe ausgewählt sind.

4. Verwendung gemäß Anspruch 3, wobei das Arzneimittel in der folgenden Gruppe aufgeführte Mikroorganismen umfaßt:
Bacteroides fragilis ss. vulgatus, Eubacterium aerofaciens, Bacteroides fragilis ss, thetaiotaomicron, Peptostreptococcus productus II, Bacteroides fragilis ss. distasonis, Fusobacterium prausnitzii, Coprococcus eutactus, Eubacterium aerofaciens III, Peptostreptococcus productus I, Ruminococcus bromii, Bifidobacterium adolescentis, Gemmiger formicilis, Bifidobacterium longum, Eubacterium siraeum, Ruminococcus torques, Eubacterium rectale III-H, Eubacterium rectale IV, Eubacterium eligens, Bacteroides eggerthii, Clostridium leptum, Bacteroides fragilis ss. a, Eubacterium biforme, Bifidobacterium infantis, Eubacterium rectale III-F, Coprococcus comes, Bacteroides capillosus, Ruminococcus albus, Eubacterium formicigenerans, Eubacterium hallii, Eubacterium ventriosum I, Fusobacterium russii, Ruminococcus obeum, Eubacterium rectale II, Clostridium ramosum I, Lactobacillus leichmanii, Ruminococcus callidus, Butyrivibrio crossotus, Acidaminococcus fermentans, Eubacterium ventriosum, Bacteroides AR, Bacteroides fragilis, ss. fragilis, Coprococcus catus, Eubacterium hadrum, E. cylindroides, E. ruminantium, Eubacterium CH-1, Staphylococcus epidermidis, Peptostreptococcus BL, Eubacterium limosum, Bacteroides praeacutus, Bacteroides L, Fusobacterium mortiferum I, F. naviforme, Clostridium innocuum, C. ramosum, Propionibacterium acnes, Ruminococcus flavefaciens, Ruminococcus AT, Peptococcus AU-1, Eubacterium AG, -AK, -AL, -AL-1, -AN; Bacteroides fragilis ss. ovatus, -ss. d, -ss. f; Bacteroides L-1, L-5; Fusobacterium nucleatum, F. mortiferum, Escherichia coli, Streptococcus morbillorum, Peptococcus magnus, Peptococcus G, -AU-2; Streptcococcus intermedius, Ruminococcus lactaris, Ruminococcus CO, Gemmiger X, Coprococcus BH, -CC; Eubacterium tenue, Eubacterium ramulus, Eubacterium AE, -AG-H, -AG-M, -AJ, -BW-1, Bacteroides clostridiiformis ss. clostridiiformis, B. coagulans, B. oralis, B. ruminicola ss. brevis, -ss. ruminicola, Bacteroides splanchnicus, Desulfomonas pigra, Bacteroides L-4, -W-1; Fusobacterium H, Lactobacillus G und Succinivibrio A.

5. Verwendung gemäß einem der Ansprüche 1 bis 4, wobei die chronische Störung eine Magen-Darm-Störung ist, die aus Colon irritabile, Colon spasticum, Colitis ulcerosa, Colitis mucosa, kollagener Colitis, Crohn-Krankheitc, Darmentzündung, mikroskopischer Colitis, Antibiotika-Colitis, idiopathischer oder einfacher Verstopfung, Divertikulose, AIDS-Enteropathie, übermäßigem Bakterienwachstum im Dünndarm, einheimischer Sprue, Polyoosis coli, Darmpolypen, chronischem, idiopatchischem Pseudoobstruktionssyndrom und chronischen Darminfektionen ausgewählt ist.

6. Verwendung gemäß Anspruch 5, wobei die chronische Darminfektion durch Bakterien, Viren, Einzeller oder Pilze verursacht ist.

7. Verwendung gemäß einem der Ansprüche 1 bis 4, wobei die chronische Störung eine Leberstörung wie etwa primär biliäre Leberzirrhose, primär sklerosierende Cholangitis, Fettleber oder kryptogene Zirrhose ist.

8. Verwendung gemäß einem der Ansprüche 1 bis 4, wobei die chronische Störung eine Gelenkstörung wie etwa rheumatoide Arthritis, eine nicht-rheumatoide Arthritis, Bechterew-Krankheit oder Reiter-Syndrom ist.

9. Verwendung gemäß einem der Ansprüche 1 bis 4, wobei die chronische Störung eine immunvermittelte Störung wie etwa Glomerulonephritis, hämolytisch-urämisches Syndrom, juveniler Diabetes mellitus, gemischte Kryoglobulinämie, Polyarteriitis nodosa, familiäres Mittelmeerfieber, Amyloidose, Sklerodermie, systemischer Lupus erythematodes oder Behcet-Syndrom ist.

10. Verwendung gemäß einem der Ansprüche 1 bis 4, wobei die chronische Störung eine neurologische Störung wie etwa Migräne, multiple Sklerose, amyotrophe Lateralsklerose, Myasthenia gravis, chronisches Müdigkeitssyndrom, Parkinson-Krankheit oder Alzheimer-Krankheit ist oder wobei die chronische Störung eine dermatologische Störung wie etwa chronische Urticaria, Akne, Dermatitis herpetiformis oder eine Vaskulitisstörung ist.

11. Verwendung gemäß einem der Ansprüche 1 bis 4, wobei die chronische Störung nekrotisierende Enterocolitis ist.

12. Verwendung gemäß einem der Ansprüche 1 bis 11, wobei die Entfernung der bestehenden Flora durch Magen-Darm-Spülung mit orthostatischer Salzlösung und Polyethylenglykollösung durchgeführt wird.

13. Verwendung gemäß Anspruch 12, wobei die Magen-Darm-Spülung durch Klistiere, orale Einnahme oder über eine Darminfusion durchgeführt wird.

14. Verwendung gemäß einem der Ansprüche 1 bis 13, wobei das Arzneimittel durch Einnahme oder durch eine Darminfusion in das Magen-Darm-System des Patienten eingeführt wird.

15. Verwendung gemäß einem der Ansprüche 1 bis 14, wobei vor dem Entfernen der bestehenden Flora eine wirksame Menge wenigstens eines Antibiotikums verabreicht wird.

## Revendications

1. Utilisation de microflore dans la préparation d'un médicament pour traiter un patient humain qui a une infection ne pouvant pas être démontrée mais qui a un désordre chronique gastrointestinal ou gastrointestinalement lié, associé à la présence dans le tractus gastrointestinal d'une microflore anormale ou d'une distribution anormale de la microflore, ledit médicament étant soit (i) une culture liquide de *Bacteroides* et de *E. coli* soit (ii) une composition de fèces homologues fraîches, une composition de fèces équivalentes cryodesséchées et reconstituées ou une composition fécale synthétique, au moins une portion de la microflore entérique existant chez le patient étant éliminée et remplacée par une quantité efficace du médicament pour restaurer chez le patient une microflore fécale normale de l'homme sain.

2. Utilisation suivant la revendication 1, dans laquelle la microflore entérique existante est éliminée pratiquement en totalité au cours du traitement.

3. Utilisation suivant la revendication 1 ou 2, dans laquelle le médicament comprend des microorganismes choisis parmi l'ensemble constitué par :
Bacteroides, Bifidobacterium, Eubacteria, Fusobacteria, Propionibacteria, Lactobacilli, Cocci anaérobies, Ruminococcus, E. coli, Gemmiger, Clostridium, Desulfomonas, et Monilia.

4. Utilisation suivant la revendication 3, dans laquelle le médicament comprend des microorganismes énumérés dans le groupe suivant :
Bacteroides fragilis ss. vulgatus, Eubacterium aerofaciens, Bacteroides fragilis ss. thetaiotaomicron, Peptostreptococcus productus II, Bacteroides fragilis ss. distasonis, Fusobacterium prausnitzii, Coprococcus eutactus, Eubacterium aerofaciens III, Peptostreptococcus productus I, Ruminococcus bromii, Bifidobacterium adolescentis, Gemmiger formicilis, Bifidobacterium longum, Eubacterium siraeum, Ruminococcus torques, Eubacterium rectale III-H, Eubacterium rectale IV, Eubacterium eligens, Bacteroides eggerthii, Clostridium leptum, Bacteroides fragilis ss. a, Eubacterium biforme, Bifidobacterium infantis, Eubacterium rectale III-F, Corpococcus comes, Bacteroides capillosus, Ruminococcus albus, Eubacterium formicigenerans, Eubacterium hallii, Eubacterium ventriosum I, Fusobacterium russii, Ruminococcus obeum, Eubacterium rectale II, Clostridium ramosum I, Lactobacillus leichmanii, Ruminococcus callidus, Butyrivibrio crossotus, Acidaminococcus fermentans, Eubacterium ventriosum, Bacteroides AR, Bacteroides fragilis ss. fragilis, Coprococcus catus, Eubacterium hadrum, E. cylindroides, E. ruminantium, Eubacterium CH-1, Staphylococcus epidermidis, Peptostreptococcus BL, Eubacterium limosum, Bacteroides praeacutus, Bacteroides L, Fusobacterium mortiferum I, F. naviforme, Clostridium innocuum, C. ramosum, Propionibacterium acnes, Ruminococcus flavefaciens, Ruminococcus AT, Peptococcus AU-1, Eubacterium AG, -AK, -AL -AL-1, -AN, Bacteroides fragilis ss. ovatus, -ss. d, -ss. f, Bacteroides L-1, L-5, Fusobacterium nucleatum, F. mortiferum, Escherichia coli Streptococcus morbillorum, Peptococcus magnus, Peptococcus G, -AU-2 Streptococcus intermedius, Ruminococcus lactaris, Ruminococcus CO, Gemmiger X, Coprococcus BH, -CC, Eubacterium tenue, Eubacterium ramulus, Eubacterium AE, -AG-H, -AG-M, -AJ, -BW-1, Bacteroides clostridiiformis ss. clostridiformis, B. coagulans, B. oralis, B. ruminicola ss. brevis, - ss. ruminicola, Bacteroides splanchnicus, Desulfomonas pigra, Bacteroides L-4, -W-1, Fusobacterium H. Lactobacillus G, et Succinivibrio A.

5. Utilisation suivant l'une quelconque des revendications 1 à 4, dans laquelle le désordre chronique est un désordre gastrointestinal choisi parmi :
le syndrome de l'intestin irritable, le côlon spasmodique, la rectocolite ulcéro-hémorragique, la colite muqueuse, la colite collagéneuse, la maladie de Crohn, la maladie inflammatoire de l'intestin, la colite microscopique, la colite associée à une antibiothérapie, la constipation idiopathique ou simple, la maladie diverticulaire, l'entéropathie du SIDA, l'hypercroissance bactérienne de l'intestin grêle, la maladie coeliaque, la polypose du côlon, les polypes du côlon, le syndrôme pseudo-obstructif idiopathique chronique et les infections entériques chroniques.

6. Utilisation suivant la revendication 5, dans laquelle l'infection entérique chronique est provoquée par une bactérie, un virus, un protozoaire ou un champignon.

7. Utilisation suivant l'une quelconque des revendications 1 à 4, dans laquelle le désordre chronique est un désordre hépatique tel que la cirrhose biliaire primaire, la cholangite sclérosante primaire, la stéatose hépatique ou la cirrhose cryptogénique.

8. Utilisation suivant l'une quelconque des revendications 1 à 4, dans laquelle le désordre chronique est un désordre articulaire tel que la polyarthrite rhumatoïde, l' arthrite non rhumatoïde, la spondylite ankylosante ou le syndrôme de Reiter.

9. Utilisation suivant l'une quelconque des revendications 1 à 4, dans laquelle le désordre chronique est un désordre d'origine immune tel que la glomérulonéphrite, le syndrôme urémique hémolytique, le diabète sucré juvénile, la cryoglobulinémie mixte, la polyartérite, la fièvre méditerranéenne familiale, l'amyloïdose, la sclérodermie, le lupus érythémateux systémique, ou le syndrôme de Behçet.

10. Utilisation suivant l'une quelconque des revendications 1 à 4, dans laquelle le désordre chronique est un désordre neurologique tel que la migraine, la sclérose en plaque, la sclérose latérale amyotrophique, la myasthénie, le syndrôme de fatigue chronique, la maladie de Parkinson ou la maladie d'Alzheimer, ou dans laquelle le désordre chronique est un désordre dermatologique tel que l'urticaire chronique, l'acné, la dermatite herpétiforme ou un désordre du type vascularite.

11. Utilisation suivant l'une quelconque des revendications 1 à 4, dans laquelle le désordre chronique est l'entérocolite nécrosante.

12. Utilisation suivant l'une quelconque des revendications 1 à 10, dans laquelle l'élimination de la flore existante est effectuée par lavage gastrointestinal avec une solution de sel orthostatique et une solution de polyéthylèneglycol.

13. Utilisation suivant la revendication 12, dans laquelle le lavage gastrointestinal est effectué par lavement, ingestion orale ou injection entérale.

14. Utilisation suivant l'une quelconque des revendications 1 à 13, dans laquelle le médicament est introduit dans le syste'me gastrointestinal du patient par ingestion ou par injection entérale.

15. Utilisation suivant l'une quelconque des revendications 1 à 14, dans laquelle une quantité efficace d'au moins un antibiotique est administrée avant l'élimination de la flore existante.
